(19) Europäisches Patentamt
European Patent Office
Office européen des brevets

(11) Publication number : **0 475 641 A1**

(12) **EUROPEAN PATENT APPLICATION**

(21) Application number : **91307948.9**

(22) Date of filing : **30.08.91**

(51) Int. Cl.$^5$ : **C07D 307/52,** C07D 333/22, C07D 207/335, C07D 213/53, C10L 1/22, C10L 1/24

(30) Priority : **10.09.90 US 580398**

(43) Date of publication of application : **18.03.92 Bulletin 92/12**

(84) Designated Contracting States : **DE ES FR GB IT**

(71) Applicant : **PETROLITE CORPORATION**
**369 Marshall Avenue**
**Saint Louis Missouri 63119 (US)**

(72) Inventor : **Weers, Jerry J.**
**309 Surf Court**
**Ballwin, Missouri 63021 (US)**
Inventor : **O'Brien, Timothy J.**
**512 Forest Green**
**St. Louis, Missouri 63119 (US)**
Inventor : **Thomasson, Catherine E.**
**2404 Westglen Farms Dr.**
**Ellisville, MO 63011 (US)**

(74) Representative : **Seaborn, George Stephen et al**
**c/o Edward Evans & Co. Chancery House**
**53-64 Chancery Lane**
**London WC2A 1SD (GB)**

(54) **Heterocyclic-amine aldehyde reaction products useful for the suppression of hydrogen sulfide.**

(57) The reaction product of a heterocyclic aldehyde (such as furfural) and a specified aminie (such as diethylene triamine) is effective as an $H_2S$ suppressant for water and hydrocarbons.

EP 0 475 641 A1

## Background of the Invention

The present invention relates to the suppression of hydrogen sulfide from hydrocarbons or water. In particular, the invention relates to such suppression by chemical means.

Hydrogen sulfide ($H_2S$) is a highly flammable and toxic gas (toxic Threshold Limit Value - Time Weighted Value = 10 ppm; Short Term Exposure Limit = 15 ppm). Hydrogen sulfide is encountered in many oil and gas formations and is a significant health hazard to oilfield and refinery workers. Accordingly, suppression of $H_2S$ in hydrocarbons, e.g., natural gas, crude oil, refined oil products, and associated water (including brine) is an important concern. Further, because of the large quantities of material to be treated, it is important to have a means of suppression that is economically efficient. Complicating the problem somewhat is the fact that some treatments will have excellent performance in one situation but poor performance in another. For instance, many treatments have undesirable side effects. Some treatments will alter the specifications of the material; e.g., viscosity, pour point, water emulsifying tendency, and some will contaminate the material with another hazardous substance; e.g., formaldehyde. Still further, the solubility of the treatment additive is sometimes a factor. All of these items often severely limit the chemistry available to scavenge hydrogen sulfide from a particular source.

One class of substances which is of particular importance with regard to hydrogen sulfide evolution is "heavy hydrocarbons" which include crude oil and refinery fractions in which at least 50 weight percent of the fraction has a boiling point of at least 240°C (at atmospheric pressure). Such materials include petroleum residua, grease, lube oil, gas oil, diesel fuel, and coker unit distillates. Because of conventional refining practices, $H_2S$ suppression is not currently a significant issue for grease, lube oil, or gas oil, but could be in the future if refinery operations are altered.

The use of various aldehydes which react with hydrogen sulfide has been known in the prior art for sometime. For example, US 2,426,318 discloses a method of inhibiting the corrosive action of natural gas and oil containing soluble sulfides on metals by utilizing certain aldehydes, preferably formaldehyde.

US 4,680,127 suggests using glyoxal to reduce the amount of hydrogen sulfide in hydrogen sulfide-containing dry gaseous and wet gaseous media.

US 4,515,759 discloses a process for removal of hydrogen sulfide from gas mixtures, particularly gas mixtures containing hydrocarbons, wherein the gas mixture is treated with a buffered aqueous solution of a water soluble nitrite, such as sodium nitrite.

Co-pending US Patent Application Serial No. 07/388,210 describes the suppression of hydrogen sulfide with the reaction product of an alkylenepolyamine and formaldehyde.

Co-pending US Patent Application Serial No. 07/374,427 describes the suppression of $H_2S$ with a reaction product of an amine or polyamine with an aldehyde or ketone. This broad disclosure does mention cyclic aldehydes, but it does not contain any disclosure of heterocyclic aldehydes.

Bottino, et al, Can. J. Chem., Vol. 59 (1981) p. 1205-1207 discloses the reaction product of furfural (2-furancarboxaldehyde) and 2-pyrrolecarboxaldehyde with butyl amine, but does not suggest the use of these materials as $H_2S$ scavengers. The article is merely a report of the type of structure formed by the reaction of these aldehydes and simple primary amines.

Robertson, J. Org. Chem., Vol. 25 (1960), p. 47-49, shows the reaction sequence used by Bottino, supra.

Hoyer, Z. Anorg. Allgem. Chem., 336(3-4), 192-6 (1965), [C. Abs. 63-9434d]; Hoyer, Z. Anorg. Allgem. Chem., 5(6), 231-2 (1965), [C. Abs. 63-16150e]; and Gallagher, J. Inorg. Nucl. Chem., (1969), 31(5), 1449-58, [C. Abs. 70-120672n] discloses the reaction product of furfural, 2-pyridinecarboxaldehyde or 2-thiophenecarboxaldehyde with ethylene diamine (1,2-diaminoethane) but do not mention the suppression of $H_2S$. These articles report the preparation of metal complexes (Zn, Co, Cu, and Ni) and the hydrolysis of such complexes.

## Summary of the Invention

The invention concerns compounds corresponding to the reaction product of a heterocyclic aldehyde and an organic primary amine, preferably an amine having at least three amino nitrogens, at least two of which are primary amino.

The compounds of the invention are useful in the suppression of hydrogen sulfide evolution from hydrocarbons or water. The compounds are particularly effective when applied to heavy hydrocarbons which consist of or which are derived from heavy crude oils.

## Cautions

Although this invention concerns hydrogen sulfide "suppression", it is important to understand that no composition can be certain to remove all hazardous levels of hydrogen sulfide under all circumstances. That

is, hydrogen sulfide may appear in hazardous concentrations after a treatment that in similar circumstances had successfully suppressed hazardous concentrations. It is important that all potential sources of hydrogen sulfide be monitored and that any suppression means not be relied on as infallible.

## Detailed Description of the Invention

In this specification and claims, numerical values are not critical unless otherwise stated. That is, the numerical values may be read as it they were prefaced with the word "about" or "substantially".

The compounds of the instant invention are imines and are conveniently prepared by reacting together a heterocyclic aldehyde and an organic primary amine.

The heterocyclic aldehydes are generally of the formula

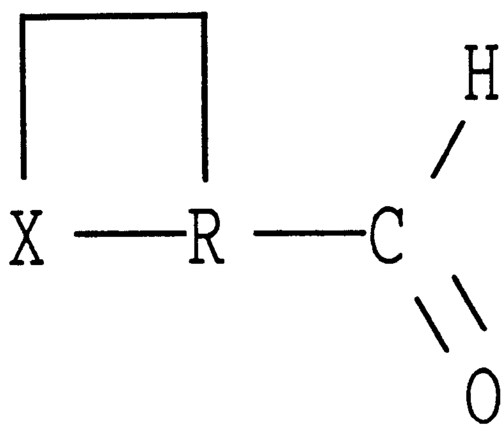

(I)

or

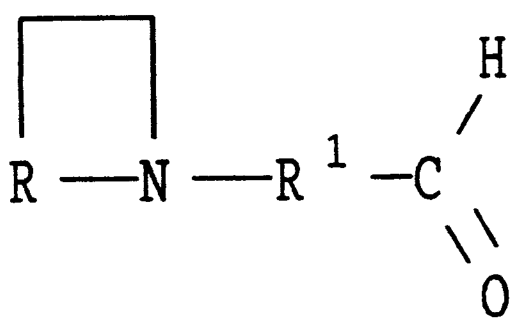

(II)

wherein X is N, O, or S, preferably O or S, more preferably O; R is an organic moiety having up to 12 carbon atoms and forming a 5 or 6 membered ring with X or N, desirably a hydrocarbon moiety having up to 8 carbon atoms, more desirably having up to 6 carbon atoms; and $R^1$ is a hydrocarbon or substituted hydrocarbon moiety having 1 to 6, desirably 1 to 4, and more desirably 1 to 2 carbon atoms. The heterocyclic aldehydes of Formula (I) are preferred to those of Formula (II).

An exemplary aldehyde is furfural (2-furancarboxaldehyde)

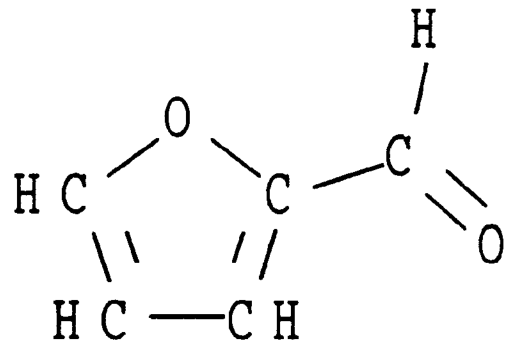

(III)

other useful aldehydee include 2-thiophenecarboxaldehyde, 3-thiophenecarboxaldehyde, 2-pyridinecarboxaldehyde, 3-pyridinecarboxaldehyde, pyrrole-2-carboxaldehyde, and pyrrole-3-carboxaldehyde.

In general, it is preferred that the aldehyde have a low molecular weight per aldehyde moiety. Compounds made from aldehydes with high ratios of molecular weight to number of aldehyde moieties will not perform as well as those with lower ratios.

The organic primary amines can be generally any organic primary or secondary amine that does not contain a moiety which is antagonistic to the reaction with the aldehyde or the suppression of $H_2S$. However, the invention excludes butyl amine if the aldehyde is furfural or 2-pyrrolecarboxaldehyde, and excludes ethylene diamine (1,2-diaminoethane) if the aldehyde is furfural, 2-pyridinecarboxaldehyde, or 2-thiophenecarboxaldehyde. Desirable amines include (1) primary and secondary (preferably primary) amines having hetero atom (preferably oxygen) substitution in the carbon chain and (2) amines having at least three amino nitrogens, at least two (or at least three) of which are primary amino. This latter preferred group includes polyaminee such as N-(2-aminoethyl)-1,2-diamino ethane (diethylene triamine), triethylene tetramine, and tetraethylene pentamine.

One class of particularly preferred amines includes those of the formula

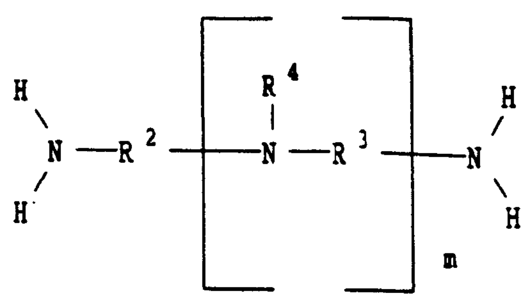

(IV)

wherein $R^2$ and each $R^3$ are independently alkylene or oxyalkylene moieties having 1 to 12, desirably 1 to 3, preferably 2 carbon atoms; each $R^4$ is independently H or an alkyl moiety having 1 to 12 carbon atoms, desirably H or an alkyl moiety having 1 or 2 carbon atoms, preferably H; and m is 1 to 4, preferably 1 or 2, more preferably 1. Any of $R^2$, $R^3$, or $R^4$ may contain hetero atoms such as oxygen. Two $R^4$ groups may be combined, for example:

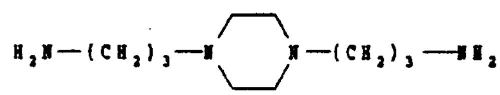

An exemplary compound according to this formula is that wherein m is 1, $R^2$ and $R^3$ are $C_2$, and $R^4$ is H; N-(2-aminoethyl)-1,2-diaminoethane (diethylene triamine).

Another class of particularly preferred amines include polyamines of the formula

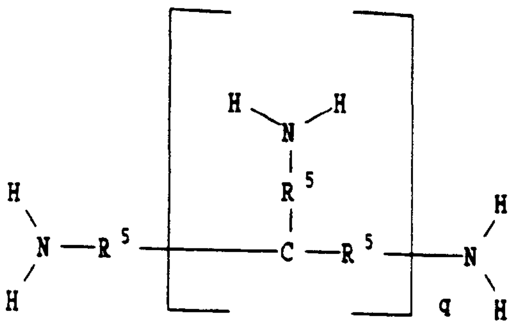

(V)

wherein each $R^5$ is independently an alkylene or oxyalkylene moiety having 1 to 5, preferably 1 to 4 carbon atoms; and q is 1 to 4, desirably 1 or 2, preferably 1. An exemplary compound of this formula is that wherein q is 1, one $R^5$ is $C_4$, one $R^5$ is $C_3$, and one $R^5$ is $C_1$ (1,8-diamino-4-aminomethyl-octane).

In general, it is preferred that the amines have a low molecular weight per primary amine moiety. Compounds made from amines with high ratios of molecular weight to number of primary amine moieties will not perform as well as those with lower ratios.

The aldehyde and amine are desirably reacted together under conventional conditions (mixing at room temperature or with mild heat) to yield an imine. The water of reaction is desirably removed by distillation (preferably vacuum distillation). The imine will have the general formula

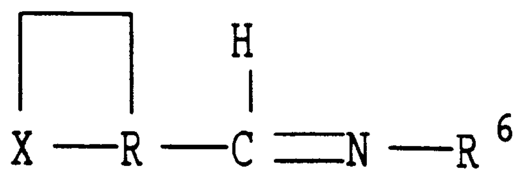

(VI)

or

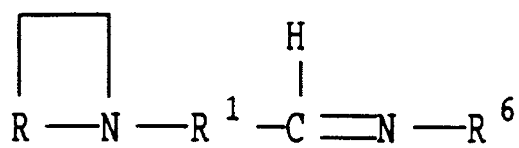

(VII)

wherein R and $R^1$ are as defined above and $R^6$ is the residue of the amine.

It should be recognized that the amine has more than one primary amine function and the resultant imine will therefore be a polyimine and the stoichiometry of the reactants should be adjusted accordingly. Although operable, it is preferred that the aldehyde not also be polyfunctional since this could result in higher molecular weight polymers which may not have appropriate solubility.

Although it is preferred that the compounds of the invention be prepared by the above discussed reaction of an amine and an aldehyde, it is possible that they could be prepared by another means. Thus, it is important only that the imine correspond to an amine/aldehyde reaction product, not that it actually is such a reaction product.

An effective amount of the above-described imine can be contacted with water or a hydrocarbon containing hydrogen sulfide to suppress the evolution of hydrogen sulfide. By "effective amount" is meant an amount sufficient to measurably reduce the quantity of evolved $H_2S$ compared to no treatment. While the precise amount of imine to be used will vary according to the specific imine compound used, the nature of the water or hydrocarbon to be treated, the level of $H_2S$ present, and the desired level of $H_2S$, in general the imines will be used at 10 to 3,000, desirably 25 to 2,000, preferably 50 to 1,000, and more preferably 100 to 500 ppm (weight basis).

While the substance to be treated may be water or a hydrocarbon, it will desirably be a liquid or solid hydrocarbon, preferably a heavy hydrocarbon. By "heavy hydrocarbon" is meant crude oil or a refinery fraction in which at least 50 weight percent of the fraction has a boiling point of at least 240°C. Thus, "heavy hydrocarbons" includes not only crude oil, but also petroleum residua, grease, lube oil, gas oil, diesel fuel, and coker unit distillates. Of these, crude oil, petroleum residua, diesel fuel, and coker unit distillates are of more significance.

The compounds of the invention are particularly useful in the control of hydrogen sulfide from petroleum residua. Residua are black viscous materials obtained as a residue from the distillation of crude oil. They may be pourable liquids (generally from distillation at atmospheric pressure) or almost solid (generally from vacuum distillation). Residua (sometimes diluted with distillates to decrease its viscosity) is encountered commercially as bunker oil, fuel oil, marine fuel oil, and asphalt. Residua contain a complex blend of components which may include a variety of sulfur compounds which can lead to the formation of hydrogen sulfide.

Of the heavy hydrocarbons, the compounds of the invention are most advantageous when applied to heavy hydrocarbons (as defined above) which originate from heavy crude oil. By "heavy crude oil" is meant crude oil having a high proportion of asphaltic and napththenic components. Such crudes will have an API gravity of $\leqq 15$, preferably $\leqq 10$. Examples of such crudes are the crude oils commonly encountered in California (USA), the West coast of Mexico, Venezuela, and the Soviet Union.

Because heavy crudes and heavy hydrocarbons made therefrom are difficult to handle and process, it is common to dilute them with other products. For instance, two or more crude oils may be blended before being refined, and two refinery products may be combined. In this regard, the invention is generally applicable to such blends. Such blends will generally be at least 5, desirably at least 10, more desirably at least 20, and preferably at least 40 percent content consisting of or derived from a heavy crude oil.

In many instances, it will be advantageous to pretreat the oil or fraction with NaOH or a similar caustic before using the method of the invention. The caustic pretreatment can enable a lower $H_2S$ level to be obtained and will help reduce the cost of the $H_2S$ suppression.

Because of the very high viscosity of many residua, it is common practice to add distilled fractions such as diesel fuel to the residua to improve its handling properties, such as to reduce its viscosity for pumpability, sprayability, and so forth. In such cases it is advantageous to add the imine compound to the distillate and then add the distillate to the residua. This will allow a uniform distribution of the imine in the residua with a minimum of mixing.

Whether or not the imine compound is first dispersed in a carrier fluid, it is important that the imine be well dispersed in the oil or fraction. Insufficient mixing will lead to higher $H_2S$ headspace concentrations. However, it should be noted that because the compounds used in this invention react very quickly with $H_2S$, mixing is less important for this invention than it is for prior art methods.

The invention will be further illustrated by the following examples. In the examples all parts and percentages are by weight unless otherwise specified.

Example 1

17.3 g of 1,8-diamino-4-aminomethyl-octane

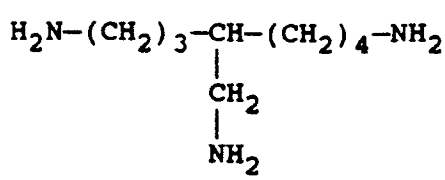

was stirred in 20 g of toluene while 28.8 g (3 equivalents) of 2-furfural were added dropwise. The mixture was heated to reflux to remove the water of reaction and a thick solution remained. Removal of the remaining toluene on a rotary evaporator left a liquid. The liquid was distilled (boiling point >230°C at 2 torr (267Pa)) to yield a viscous dark orange oil. Since decomposition during the distillation was noted, the procedure was repeated, using the rotoevaporator to remove all of the solvent (i.e., no distillation) yielding a dark, viscous oil.

Example 2

19.1 g (0.09 mole) of bis-hexamethylenetriamine [N-(6-aminohexane)-1,6-diaminohexane]

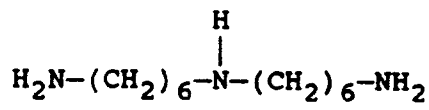

was mixed with 19.2 g (0.2 mole) of 2-furfural and 40 g of xylene (as a solvent) and heated to 50-70°C, with stirring, for one hour. The water of reaction was then distilled off into a Dean Stark Trap (water yield = 2.1 g). The mixture was cooled, yielding a dark red oil.

Example 3

0.4 mole of diethylenetriamine [N-(2-aminoethyl)-1,2-diaminoethane)

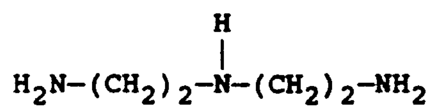

was dissolved in 53 g of Solvesso 150 aromatic solvent and 0.8 mole of 2-furfural was added dropwise with stirring. With the stirring continued, the mixture was heated to 70°C for one hour. 20 ml of xylene was added and the water removed by distillation. (the temperature in the distillation flask was allowed to reach 170°C). The product was a thick, dark oil.

## Examples 4-10

Generally following the procedures of Examples 1-3, the following reactants were reacted to produce an imine:

Example 4

aldehyde: (2) equivalents) 2-furfural
amine: Texaco D-230 amine

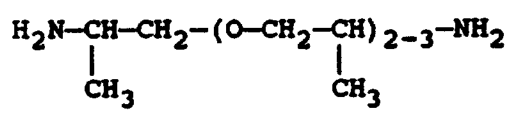

Example 5

aldehyde: (2 equivalents) 2-furfural
amine: 1,2-diaminocyclohexane

Example 6

aldehyde: (2 equivalents) furfural
amine: Du Pont Dytek A amine

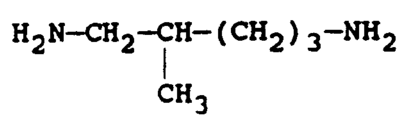

Example 7

aldehyde: 2-thiophencarboxyaldehyde

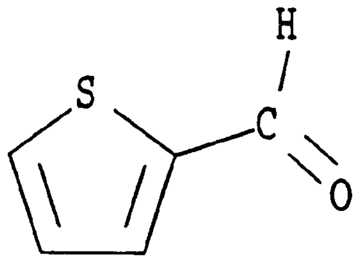

amine: butylamine
Note: compare Examples 10 and 13

Example 8

aldehyde: 3-thiophenecarboxaldehyde
amine: butylamine

Example 9

aldehyde: 2-pyridinecarboxaldehyde

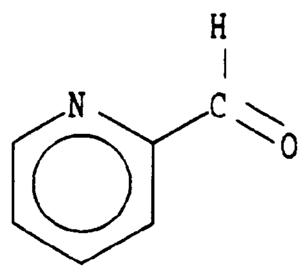

amine: butylamine
Note: compare Example 12

Example 10

aldehyde: 3-pyridinecarboxaldehyde
amine: butylamine

Example 11

A residual fuel oil containing 28% pitch derived from crude oil produced in the San Joaquin Valley (California), the remainder being pitch derived from Alaskan North Slope crude and gas oil, was sampled using a 1 liter Welker CP2-1000A sample cylinder (Welker Engineering Co., Sugar Land, Texas) in a manner so as to have no headspace (i.e., no air) in the cylinder. A treatment compound was introduced via a port on the side of the cylinder and thorough mixing achieved by five transfers to a Welker CP2-1000MA automixer. A headspace equal in volume to the liquid was created by expelling half of the sample and introducing normal air. After the headspace $H_2S$ level reached equilibrium, an $H_2S$ headspace concentration was recorded. The entire sampling, mixing, and measuring process took place at 125°-145°F (52°-63°C). Some samples were subjected to a treatment with NaOH prior to treatment with the organic compound. The data are reported in Table I.

TABLE I

| Sample | Organic Compound | Organic Compound Treatment Level (ppm) | NaOH Pretreatment (ppm Na) | Headspace $H_2S$ (ppm) |
|---|---|---|---|---|
| - 1* | --- | --- | --- | 2800 |
| - 2* | --- | --- | 70 | 850 |
| - 3* | Compound A(1) | 1500 | --- | 480 |
| - 4* | " | 500 | 70 | 250 |
| - 5* | Compound C(2) | 1500 | --- | 1200 |
| - 6* | " | 500 | 70 | 350 |
| - 7 | Example 1 | 1500 | --- | 500 |
| - 8 | " | 500 | 70 | 140 |
| - 9(3) | " | 500 | 70 | 80 |
| -10 | Example 3 | 1500 | --- | 50 |
| -11 | " | 500 | --- | 300 |
| -12 | " | 500 | 70 | 50 |
| -13(3) | " | 250 | 70 | 120 |

* Not an example of the invention.
(1) A condensate of formaldehyde and a hindered primary amine.
(2) A condensate of formaldehyde and an alkyl secondary amine.
(3) Mixed 3-4 times as long as the other samples.

Example 12

The procedure of Example 11 was repeated with a residual fuel oil containing 32% San Joaquin Valley pitch, the remainder being pitch from an Alaskan North Slope Crude oil and distillate from the two crudes. The data are reported in Table II.

TABLE II

| Sample | Organic Compound | Organic Compound Treatment Level (ppm) | NaOH Pretreatment (ppm Na) | Headspace $H_2S$ (ppm) |
|---|---|---|---|---|
| - 1* | --- | --- | --- | 800 |
| - 2* | --- | --- | 70 | 600 |
| - 3* | Compound A(1) | 1000 | 70 | 175 |
| - 4* | " | 500 | 70 | 200 |
| - 5* | Compound C(2) | 1000 | 70 | 190 |
| - 6* | " | 500 | 70 | 250 |
| - 7 | Example 1 | 1000 | 70 | 0 |
| - 8 | " | 500 | 70 | 100 |
| - 9(3) | " | 500 | 70 | 50 |
| -10 | Example 3 | 1000 | 70 | 0 |
| -11 | " | 500 | 70 | 100 |
| -12(3) | " | 250 | 70 | 40 |

* Not an example of the invention.
(1) (2) See Table I.
(3) Mixed 3-4 times as long as the other samples.

Example 13

The procedure of Examples 11 and 12 was repeated with residual fuel oils containing varying amounts of pitch from a San Joaquin Valley crude oil and the compound of Example 3. The results are reported in Table III.

TABLE III

| Sample | San Joaquin Valley Pitch (%) | Compound of Example 3 (ppm) | NaOH Pretreat (ppm Na) | $H_2S$ Headspace (ppm) |
|---|---|---|---|---|
| - 1* | 7 | --- | --- | 960 |
| - 2* | " | --- | 70 | 0 |
| - 3 | " | 50 | --- | 10 |
| - 4 | " | 25 | --- | 50 |
| - 5* | 7 | --- | --- | 625 |
| - 6* | " | --- | 85 | 400 |
| - 7 | " | 1000 | --- | 375 |
| - 8 | " | 100 | --- | 500 |
| - 9(1) | " | 50 | --- | 600 |
| -10 | " | 1000 | 70 | 10 |
| -11(1) | " | 750 | 70 | 150 |
| -12 | " | 500 | 70 | 475 |
| -13* | 7 | --- | --- | 1050 |
| -14* | " | --- | 70 | 750 |
| -13 | " | 3000 | --- | 75 |
| -14 | " | 1500 | 70 | 30 |
| -15* | 22 | --- | --- | 775 |
| -16* | " | --- | 70 | 700 |
| -17 | " | 1000 | --- | 40 |
| -18 | " | 500 | --- | 200 |
| -19 | " | 1000 | 70 | 15(2) |
| -20 | " | 500 | 70 | 60 |
| -21 | " | 250 | 70 | 100 |
| -22 | " | 125 | 70 | 225 |
| -23* | 46 | --- | --- | 1900 |
| -24* | " | --- | 70 | 1600 |
| -25 | " | 500 | 70 | 100 |
| -26(3) | " | 500 | 70 | 75 |
| -27 | " | 500 | 35 | 325 |
| -28 | " | 500 | --- | 550 |

* Not an example of the invention.
(1) Mixed twice as long as other samples.
(2) Average of two runs.
(3) Mixed three times as long as other samples.

## Claims

1. A compound corresponding to the reaction product of
   a. a heterocyclic aldehyde; and
   b. an organic primary or secondary amine, with the proviso that if the aldehyde is furfural or 2-pyrrolecarboxaldehyde, the amine is not butyl amine, and if the aldehyde is furfural, 2-pyridinecarboxaldehyde, or 2-thiophenecarboxaldehyde, the amine is not ethylene diamine.

2. The compound of Claim 1 wherein the amine contains a hetero atom in the carbon chain.

3. The compound of Claim 2 wherein the hetero atom is oxygen.

4. The compound of Claim 1 wherein the amime is an amine having at least three amino nitrogens, at least two of which are primary amino.

5. The compound of Claim 4 wherein the organic primary amine is of the formula

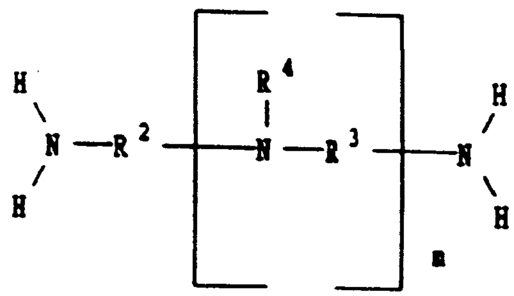

(IV)

wherein $R^2$ and each $R^3$ are individually a $C_1$ to $C_4$ alkylene or oxyalkylene moiety; each $R^4$ is individually H or a $C_1$ to $C_4$ alkyl moiety; and m is 1 to 4; or

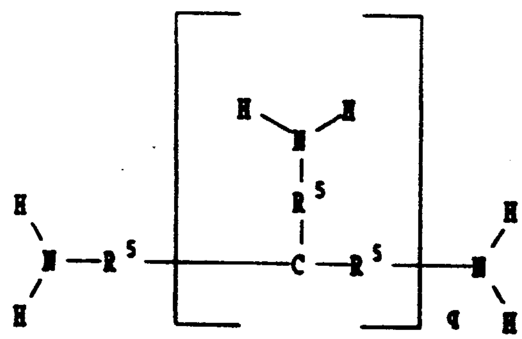

(V)

wherein each $R^5$ is individually a $C_1$ to $C_5$ alkylene or oxyalkylene moiety; and q is 1 to 4.

6. The compound of Claims 1, 2, 3, 4, or 5 wherein the heterocyclic aldehyde has the general formula

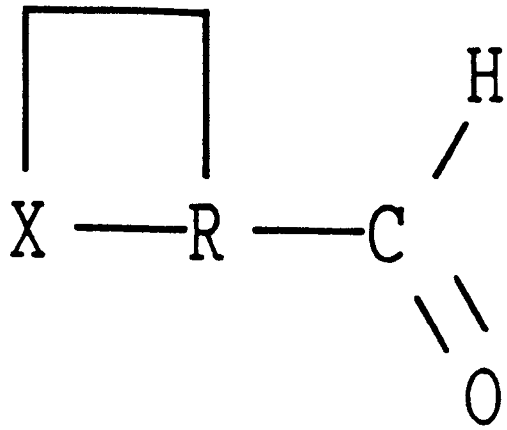

(I)

or

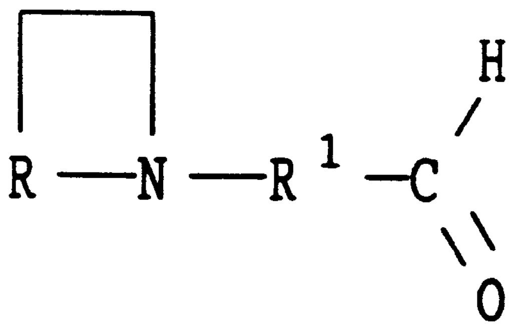

(II)

wherein X is N, O, or S; R is an organic moiety forming a 5 or 6 membered ring and having up to 12 carbon atoms; and $R^1$ is a hydrocarbon or substituted hydrocarbon moiety having 1 to 6 carbon atoms.

7. The compound of claim 5 wherein the aldehyde is 2-furfural, 3-furfural, 2-thiophenecarboxaldehyde, 3-thiophenecarboxaldehyde, 2-pyridinecarboxaldehyde, 3-pyridinecarboxaldehyde, pyrrole-2-carboxaldehyde, and pyrrole-3-carboxaldehyde.

8. The compound of Claim 7 wherein the aldehyde is 2-furfural.

9. A composition comprising a compound according to any preceding claim.

European Patent Office

# EUROPEAN SEARCH REPORT

Application Number

EP 91 30 7948

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-3 468 830 (KISS)<br>* the whole document * | 1-9 | C07D307/52<br>C07D333/22<br>C07D207/335<br>C07D213/53<br>C10L1/22<br>C10L1/24 |
| X | DE-A-2 002 491 (SOLCO BASEL AG.)<br>* the whole document * | 1,6,9 | |
| D,X | CANADIAN JOURNAL OF CHEMISTRY.<br>vol. 59, 1981, OTTAWA CA<br>pages 1205 - 1207;<br>BOTTINO ET AL.: 'A SIMPLE 1H NMR CONFORMATIONAL STUDY OF SOME HETEROCYCLIC AZOMETHINES'<br>* the whole document * | 1,6,9 | |
| D,X | JOURNAL OF INORGANIC AND NUCLEAR CHEMISTRY.<br>vol. 31, no. 5, January 1969, OXFORD GB<br>pages 1449 - 1458;<br>COAKLEY ET AL.: 'STUDIES OF METAL CHELATES FORMED FROM DERIVATIVES OF 2-THIOPHENEALDEHYDE AND FURFURAL'<br>* the whole document * | 1-9 | |
| | | | **TECHNICAL FIELDS SEARCHED (Int. Cl.5)** |
| | | | C07D<br>C10L |

The present search report has been drawn up for all claims

| Place of search | Date of completion of the search | Examiner |
|---|---|---|
| THE HAGUE | 05 DECEMBER 1991 | DE LA MORINERIE |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)